(19) Europäisches Patentamt
European Patent Office
Office européen des brevets

(11) Publication number: 0 164 204 A1

# (12) EUROPEAN PATENT APPLICATION

(21) Application number: 85302902.3

(22) Date of filing: 25.04.85

(51) Int. Cl.[4]: A 61 K 31/505, C 07 D 401/12, C 07 D 403/12, C 07 D 417/12, C 07 D 239/42, C 07 D 401/04, C 07 D 403/04, C 07 D 417/04

(30) Priority: 12.05.84 GB 8412184
08.01.85 GB 8500417

(43) Date of publication of application: 11.12.85 Bulletin 85/50

(84) Designated Contracting States: AT BE CH DE FR GB IT LI LU NL SE

(71) Applicant: FISONS plc, Fison House Princes Street, Ipswich Suffolk IP1 1QH (GB)

(72) Inventor: Hashimoto, Masashi, 1-6-17 Nakayama-Satsukidai, Takarzuka (JP)
Inventor: Robinson, David Hulme, 11 Countrymans Way, Stepshed Leicestershire (GB)

(74) Representative: Craig, Christopher Bradberry et al, Fisons plc 12 Derby Road, Loughborough Leicestershire LE11 0BB (GB)

## (54) Novel pharmaceutically useful pyrimidines.

(57) There are described compounds of formula I,

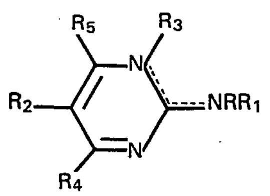

I

in which one of the groups R and $R_3$ has no significance and the other is hydrogen, phenyl or alkyl Cl to 6 optionally substituted by phenyl, and when $R_3$ has no significance R can additionally represent alkanoyl Cl to 6,

one of the bonds --- is a double bond and the other is a single bond,

$R_1$, and at least one of $R_2$, $R_4$ and $R_5$, may be the same or different and are selected from a pyridinyl, pyridimidinyl, thiazolyl, pyrazinyl, pyridazinyl, imidazolyl and phenyl ring, which rings may optionally be substituted by one or more of the groups halogen, $-COOR_5$, $-COR_5$, -CN, $-CONH_2$, $-SO_2NR_5R_6$, $-NR_5R_6$, $-OR_5$ or alkyl Cl to 6 which latter is optionally substituted by fluorine,

$R_5$ and $R_6$, which may be the same or different, each represent hydrogen or alkyl Cl to 6 optionally substituted by mono- or di-alkyl (Cl to 6) amino,

and the remainder of $R_2$, $R_4$ and $R_5$ are selected from hydrogen, hydroxy, alkyl Cl to 6 and alkoxy Cl to 6 in addition to the significances given above,

or $R_1$, and one of $R_4$ and $R_5$ have the significances given above and anadjacent pair of $R_2$, $R_4$ and $R_5$ together form a -CH=CH-CH=CH- chain,

or a pharmaceutically acceptable acid addition salt thereof.

Also described are processes for making certain of the compounds of formula I, formulations containing the compounds of formula I and their use as pharmaceuticals.

EP 0 164 204 A1

NOVEL PHARMACEUTICALLY USEFUL PYRIMIDINES

This invention relates to new compounds, methods for their preparation and compositions containing them.

According to the invention we provide the use as a pharmaceutical of a compound of formula I,

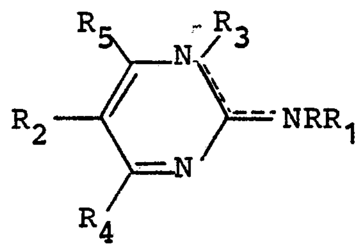

I

in which one of the groups R and $R_3$ has no significance and the other is hydrogen, phenyl or alkyl C1 to 6 optionally substituted by phenyl, and when $R_3$ has no significance R can additionally represent alkanoyl C1 to 6,

one of the bonds --- is a double bond and the other is a single bond,

$R_1$, and at least one of $R_2$, $R_4$ and $R_5$, may be the same or different and are selected from a pyridinyl, pyrimidinyl, thiazolyl, pyrazinyl, pyridazinyl, imidazolyl and phenyl ring, which rings may optionally be substituted by one or more of the groups halogen, $-COOR_5$, $-COR_5$, $-CN$, $-CONH_2$, $-SO_2NR_5R_6$, $-NR_5R_6$, $-OR_5$ or alkyl C1 to 6 which latter is optionally substituted by fluorine,

$R_5$ and $R_6$, which may be the same or different, each represent hydrogen or alkyl C1 to 6 optionally substituted by mono- or di-alkyl (C1 to 6) amino,

and the remainder of $R_2$, $R_4$ and $R_5$ are selected from hydrogen, hydroxy, alkyl C1 to 6 and alkoxy C1 to 6 in addition to the significances given above,

or $R_1$, and one of $R_4$ and $R_5$ have the significances given above and an adjacent pair of $R_2$, $R_4$ and $R_5$ together form a -CH=CH-CH=CH- chain,

or a pharmaceutically acceptable salt thereof.

According to the invention we also provide as new compounds those compounds of formula I in which $R_2$ and $R_4$ do not together form a chain -CH=CH-CH=CH-, and the pharmaceutically acceptable salts thereof.

According to the invention we also provide a process for the production of a new compound of formula I, or a pharmaceutically acceptable salt thereof, which comprises

a) reaction of a compound of formula II,

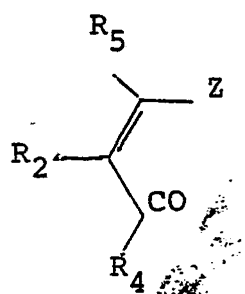

II

in which $R_2$, $R_4$ and $R_5$ are as defined above, and Z is a good leaving group,

with a compound of formula III,

$$R_1\text{-NRC(=NR}_3\text{)NH}_2 \qquad \text{III}$$

or a salt thereof,

in which R, $R_1$ and $R_3$ are as defined above,

b) production of a compound of formula I in which R is alkanoyl $C_1$ to 6 by alkanoylation of a corresponding compound of formula I in which one of R and $R_3$ is hydrogen,

c) production of a compound of formula I in which one of R and $R_3$ is alkyl C1 to 6 optionally substituted by phenyl, by alkylation or phenylalkylation of a corresponding compound of formula I in which one of R and $R_3$ is hydrogen,

d) production of a compound of formula I carrying a -COOH substituent by hydrolysis of a corresponding compound of formula I carrying a -COOalkyl or $-CONH_2$ substituent,

e) production of a compound of formula I carrying an -OH substituent by removal of an alkyl group from a corresponding compound of formula I carrying an alkoxy substituent, or

f) production of a compound of formula I in which at least one of $R_2$, $R_4$ and $R_5$ is alkoxy optionally

substituted by mono-or di-alkylamino, by reaction of a corresponding compound of formula I in which at least one of $R_2$, $R_4$ and $R_5$ is hydroxy, with an optionally mono-or di-alkylamino substituted alkylating agent,

and if desired or necessary converting the resulting compound of formula I to pharmaceutically acceptable salt thereof or vice versa.

Process a) may be carried out in a solvent which is also a base, e.g. pyridine, and may be carried out at temperatures ranging from $0^o$ to the reflux temperature of the reaction medium, e.g. at about $115^o$. The good leaving group Z may be, for example, a dialkylamino, hydroxy or alkoxy, e.g ethoxy, group. When $R_4$ in the compound of formula II is alkoxy the group $R_4$ in the product compound is -OH.

The alkanoylation of process b) may be carried out using a suitable alkanoylating agent, e.g. an appropriate anhydride such as acetic anhydride, in pyridine. The reaction may be carried out at a temperature of from about 0 to $50^oC$.

Process c) may be carried out in a solvent which is inert under the reaction conditions, e.g. diethyl ether, acetone or acetonitrile. The alkylating agent may be, for example, an alkyl halide, e.g. methyl iodide, or a phenylalkyl halide, e.g. benzyl bromide. The reaction may

be carried out in the presence of a base, eg sodium hydride when substitution in position R is required.

The hydrolysis of process d) may be carried out using a base, e.g. sodium hydroxide, in a water miscible solvent, e.g. ethanol or glyme.

Process e) may be carried out using conventional ether cleavage techniques, eg a mixture of acetic acid and hydrobromic acid or sodium sulphide in a suitable solvent, eg N-methylpyrrolidone. The reaction is preferably carried out at an elevated temperature, eg of from about $80^{o}$ to $100^{o}C$.

The alkylation of process f) may be carried out using conventional alkylation conditions, e.g. where $R_4$ in the product compound is to be methoxy using diazomethane in a solvent which is inert under the reaction conditions, e.g. N,N-dimethylformamide. Alternatively the compound of formula I in which one of $R_2$, $R_4$ and $R_5$ is hydroxy may be reacted with an optionally substituted alkyl halide, e.g. in the presence of a strong base.

The compounds of formula I may be recovered from their reaction mixture using conventional techniques which are known per se.

The starting materials for the above processes are either known or they may be made from known compounds using conventional techniques known per se.

Pharmaceutically acceptable salts of the compounds of formula I include acid addition salts with pharmaceutically acceptable organic or inorganic anions, e.g. the chloride, sulphate, maleate or tartrate anions.

The compounds of formula I, and the pharmaceutically acceptable salts thereof, are useful because they possess pharmacological activity in animals; in particular they are useful because they possess immunoregulant activity, e.g. in the test set out in Example A. Thus the new compounds are indicated for use in the elevation of depressed immune responses associated with therapy, e.g. with cytotoxic drugs, immunosuppressive drugs or by radiotherapy or surgery. The compounds thus render the patient less susceptible to immunosuppressive side effects such as secondary infectious episodes or bone marrow depression. The compounds may be applied as an adjunct to existing therapy.

The compounds are also indicated for elevation of depressed immune responses associated with secondary immunodeficiency disease (AIDS, neoplastic disease) and in infectious diseases mediated by viral, bacterial, fungal or metazoan parasitic agents. Thus the compounds may be applied alone or with an anti-infective agent. The compounds are further indicated for use in thermal injuries, surgery (post-operative stress), wound healing

and immunodeficiencies associated with ageing. The compounds are also indicated to modulate aberrant immunoregulatory pathways as seen in rheumatoid arthritis and systemic lupus erythematosis.

For the above mentioned uses the dosage administered will, of course, vary with the compound employed, the mode of administration, the treatment desired (e.g. topical, parenteral or oral) and the disease indicated. However, in general, satisfactory results are obtained when the compounds are administered at a dosage of from 0.1 to 200mg per kg of animal body weight in the test set out in Example A. For man the indicated total daily dosage is in the range of from 1mg to 1000mg and preferably from 10mg to 500mg, which may be administered, for example twice weekly, or in divided doses from 1 to 6 times a day or in sustained release form. Thus unit dosage forms suitable for administration, e.g. oesophageally, comprise from 2mg to 500mg, and preferably 1mg to 500mg of the compound preferably admixed with a solid or liquid pharmaceutically acceptable diluent, carrier or adjuvant.

We prefer $R_1$ and those of $R_2$, $R_4$ and $R_5$ which represent rings to be different. We also prefer at least one of $R_1$ and $R_2$ to be a substituted ring. Thus when $R_2$, $R_4$ or $R_5$ is a benzene ring we prefer the ring to carry a substituent in a position para to the pyrimidine

group. When $R_1$ is a benzene ring we prefer the ring to be unsubstituted or to carry a substituent para to the group -NR-. Specific substituents on the benzene (or other rings) are halogen, e.g. chlorine; hydroxy; alkoxy C1 to 6, e.g. $-OCH_3$; -CN; -COOH; $-COOCH_3$; $-COCH_3$; $-SO_2N(CH_3)_2$; $-N(CH_3)_2$; $-CH_3$ or $-OCH_2CH_2N(C_2H_5)_2$. We prefer $R_3$ to have no significance. Specific values for R are H, $-CH_3$, $-C_2H_5$, $-CH_2CH_2CH_3$, $-CH(CH_3)_2$, acetyl, 2-methylpropanoyl, phenyl or benzyl. We prefer $R_4$ to be hydrogen.

We particularly prefer $R_2$ to be a para substituted, e.g. a para alkoxy (methoxy) substituted, benzene ring, $R_4$ and $R_5$ to be hydrogen, $R_3$ to have no significance, R to be alkyl C1 to 4, e.g. methyl, or alkanoyl C2 to 4, e.g. acetyl, and for $R_1$ to be phenyl and preferably unsubstituted phenyl.

According to our invention we also provide a pharmaceutical composition comprising (preferably less than 80%, and more preferably less than 50% by weight) of a compound of formula I, or a pharmaceutically acceptable salt thereof, in combination with a pharmaceutically acceptable adjuvant, diluent or carrier. Examples of suitable adjuvants, diluents or carriers are: for tablets, capsules and dragées; microcyrstalline cellulose, calcium

phosphate, diatomaceous earth, a sugar such as lactose, dextrose or mannitol, talc, stearic acid, starch, sodium bicarbonate and/or gelatin; for suppositories, natural or hardened oils or waxes; and for inhalation compositions, coarse lactose. The compound of formula I, or the pharmaceutically acceptable salt thereof, preferably is in a form having a mass median diameter of from 0.01 to 10 microns. The compositions may also contain suitable preserving, stabilising and wetting agents, solubilisers, sweetening and colouring agents and flavourings. The compositions may, if desired, be formulated in sustained release form. We prefer compositions which are designed to be taken oesophageally and to release their contents in the gastrointestinal tract.

Compounds of formula I in which $R_2$, $R_4$ or $R_5$ is hydroxy can also exist in tautomeric keto forms which are also encompassed within the present invention.

The invention is illustrated, but in no way limited by the following Examples.

Example 1

5-(4-Methoxyphenyl)-N-phenylpyrimidine-2-amine

2-(4-Methoxyphenyl)-3-dimethylaminoacrolein (10.0g) and phenylguanidine bicarbonate (10.0g) were heated at reflux in pyridine (100ml) for 17 hours. The solvent was removed *in vacuo* and the resulting residue was suspended

in chloroform and washed with 2N sodium hydroxide solution followed by water. The suspended solids (9g) were collected by filtration and recrystallised from ethyl acetate to give the title compound as a white solid (5.4g) melting point 216-9°.

| | |
|---|---|
| Found: | C73.61, H5.45, N15.00% |
| $C_{17}H_{15}N_3O$ requires | C73.65, H5.42, N15.16% |

Example 2

1,2-Dihydro-5-(4-methoxyphenyl)-1-methyl-2-phenyliminopyrimidine

Methyl iodide (19.2 g) was added to a solution of 5-(4-methoxyphenyl)-2-phenylaminopyrimidine (1.5 g) in N,N-dimethylformamide (150ml) and the mixture was stirred for 22 hours at 100°. After evaporation, the residue was dissolved in chloroform and the solution was washed with 3% potassium hydroxide solution. Drying over magnesium sulphate and evaporation gave the title compound (1.42 g) as an orange solid. mp 124-125°.

Anal. Calcd. for $C_{18}H_{17}N_3O$; C:71.46, H:5.37, N:13.16, Found: C:71.55, H:5.49, N:13.23.

Example 3

5-(4-Methoxyphenyl)-N-methyl-N-phenylpyrimidine-2-amine

A suspension of 60% sodium hydride (8.7 mg) in N,N-dimethyl-formamide (5 ml) was added to a solution of

5-(4-methoxyphenyl)-N-phenylpyrimidine-2-amine (100 mg) in N,N-dimethylformamide (8 ml) and the mixture was stirred for 15 minutes at 0°. Methyl iodide (51 mg) was added and the solution was stirred for 30 minutes at 0°. The solvent was evaporated off and ethanol was added to the residue. The resulting precipitate was collected by filtration to give the title compound (66 mg) as a white solid. mp 149-150°.

Example 4

N-Acetyl-5-(4-methoxyphenyl)-N-phenylpyrimidine-2-amine

5-(4-Methoxyphenyl)-N-phenylpyrimidine-2-amine (1.0 g) was suspended in acetic anhydride (50 ml) and the mixture was stirred for 5 hours at 90°. The resulting clear solution was concentrated *in vacuo*. The residue was dissolved in chloroform (150 ml) and the solution was washed with 5% sodium bicarbonate solution followed by water. Drying over magnesium sulphate and evaporation gave an oily residue, which was applied to a silica gel column and eluted with a mixture of chloroform and methanol to afford the title compound as a white solid (0.98 g). mp 90-91°.

Anal. Calcd. for $C_{19}H_{17}N_3O_2$, C:71.46, H:5.37, N:13.16,
Found: C:71.55, H:5.49, N:13.23.

Using the process of this Example there was also

made:-

5-(4-Methoxyphenyl)-N-(2-methylpropanoyl)-N-phenyl-pyrimidine-2-amine. mp 118.5-120°.

Example 5

4-[5-(4-Methoxyphenyl)pyrimidin-2-yl]aminobenzoic acid

Methyl 4-[5-(4-methoxyphenyl)-pyrimidin-2-yl]aminobenzoate (2.0 g) was dissolved in the mixture of dioxane (150 ml), water (60 ml) and 1N sodium hydroxide solution (18 ml) at 100° and the solution was stirred for 18 hours at room temperature. 1N Hydrochloric acid (18 ml) was added to the reaction mixture. The resulting precipitate was collected by filtration and washed with water and methanol successively, dried over phosphorous pentoxide under reduced pressure to give the title compound (1.65 g) mp > 300°. Mass m/z 321 ($M^+$).

Example 6

5-(4-Hydroxyphenyl)-N-(4-hydroxyphenyl)pyrimidine -2-amine

5-(4-Methoxyphenyl)-N-(4-methoxyphenyl)pyrimidine -2-amine (1.0 g) was heated at reflux in a mixture of acetic acid (80 ml) and 47% hydrobromic acid (80 ml) for 3 hours. The solvent was evaporated off and the resulting residue was suspended in water and neutralised to pH5 with sodium bicarbonate. The suspended solid was collected by filtration and dissolved in ethyl acetate. The solution

was subjected to silica gel column chromatography and eluted with ethyl acetate to give a yellow solid. The solid was washed with a small amount of acetone to afford the title compound (0.5 g) as pale yellow crystals. mp 246-247.5°. Mass m/z 279 ($M^+$).

Example 7

5-(4-Hydroxyphenyl)-N-phenylpyrimidine-2-amine

5-(4-Methoxyphenyl)-N-phenylpyrimidine-2-amine (2.0 g) and sodium sulphide (2.81 g) were heated at 140° in N-methyl-2-pyrrolidone (10 ml) for 24 hours. After cooling, the mixture was acidified with 0.1N hydrochloric acid. The resulting precipitate was collected by filtration to give a yellow solid. The solid was washed with carbon disulphide to give the title compound (0.94 g) as a white solid. mp 205-206°. Mass m/z 263 ($M^+$).

Example 8

N-Phenyl-4-(4-methoxyphenyl)pyrimidine-2-amine

p-Methoxy acetophenone (5.65 g) and N,N-dimethylformamide dimethylacetal (8 ml) were heated under reflux for 2 days. After evaporation, the residue was washed with ether to give 1-(N,N-dimethylamino)-3-(4-methoxyphenyl)-propenone (5.81 g). The propenone (4.0 g) and phenylguanidine carbonate (3.89 g) were heated under reflux in pyridine (50 ml) for 12 hours. The solvent was evaporated off and the residue was treated with chloroform

and water. The organic layer was collected and the solvent was evaporated to give a yellow solid, which was subjected to a silica gel column chromatography, eluted with chloroform to afford the title compound (2.45 g) as white crystals. mp 145-146°. Mass m/z 277 ($M^+$).

Anal. Calcd for $C_{17}H_{15}N_3O$, C:73.63, H:5.45, N:15.15.

Found: C:73.74, H:5.59, N:15.24.

<u>Example 9</u>

<u>N-Phenyl-5-(4-pyridyl)-pyrimidine-2-amine</u>

3-Hydroxy-2-(4-pyridyl)propenal (3.0g), prepared from -picoline by the method described in the literature (Coll. Czech. Chem. Comm., <u>28</u>, 863 (1963)), and phenylguanidine carbonate (4.0g) were heated at reflux in pyridine (30ml) for 12 hours. The solvent was evaporated off and the resulting residue was dissolved in conc. hydrochloric acid. The solution was washed with chloroform and neutralised with 50% potassium hydroxide. The resulting precipitate was collected to afford a white solid. The solid was recrystallised from methanol to give the title compound (0.96g) as white crystals mp. 244-245.5°.

<u>Example 10</u>

<u>5-(4-Methoxyphenyl)-2-phenylamino-4(1H)-pyrimidinone</u>

Ethyl 3-hydroxy-2-(4-methoxyphenyl)acrylate (14.00g) and phenylguanidine carbonate (10.47g) were heated under

reflux in pyridine (280ml) for 17 hours. After the reaction mixture was cooled, the resulting precipitate was collected. The crude product was suspended in hot ethyl acetate and collected after cooling to afford the title compound (10.8g) as white crystals. mp 250°.

Analysis:

| | |
|---|---|
| Calcd for $C_{17}H_{15}N_3O_2$, | C:69.61, H:5.15, N:14.33. |
| Found: | C:70.02, H:5.27, N:14.58 |

Example 11

4-Methoxy-5-(4-methoxyphenyl)-N-phenylpyrimidine -2-amine

A solution of diazomethane in ether, prepared from p-toluene-sulfonylmethylnitrosamide and potassium hydroxide according to the literature (Org. Syn., Coll. Vol. 4, 2550 (1963)), was added to a solution of 5-(4-methoxyphenyl)-2-phenylamino-4(1H)-pyrimidinone (3.0g) in N,N-dimethylformamide (200ml). The reaction mixture was stirred overnight and acetic acid was added to decompose diazomethane. The solvent was evaporated and the residue was dissolved in chloroform and washed with an aqueous solution of sodium bicarbonate and brine. Drying over magnesium sulphate and evaporation gave a crude product, which was applied to a silica gel column chromatography eluting with a mixture of chloroform and methanol to afford the title compound (1.3g) as white

crystals. mp. 208-209°. MS m/z 307 ($M^+$).

Example 12

5-(4-Methoxyphenyl)-N-(2-pyrimidyl)pyrimidine-2-amine

A mixture of 2-chloropyrimidine(3.5g) and guanidine carbonate (2.73g) in N,N-dimethylformamide(30 ml) was heated at reflux for 36 hours. After evaporation of the solvent, the residue was dissolved in methanol (15ml) and a 28% solution of sodium methoxide in methanol (6.5ml) was added. The resulting precipitate was removed by filtration and the filtrate was concentrated to give a brownish residual oil, which was dissolved in pyridine (50ml) and, after 2-(4-methoxyphenyl)-3-dimethylamino-acrolein (6.28g) was added, the mixture was heated at reflux overnight. The solvent was evaporated and methanol was added. The resulting precipitate was collected and purified by dissolution in conc. hydrochloric acid and neutralization with potassium carbonate. The precipitated solid was collected, washed with 0.1N sodium hydroxide and dissolved in chloroform. After treatment with activated charcoal, chloroform was evaporated and the residue was washed with methanol to give the title compound (0.91g) as white crystals. mp; 200-202°.

Example 13

5-[4-(2-Diethylaminoethoxy)phenyl]- N-methyl-N-phenylpyrimidine-2-amine

A mixture of 5-(4-methoxyphenyl)-N-methyl-N-phenyl pyrimidine-2-amine (1.0g) and sodium sulfide (1.34g) in N-methyl-2-pyrrolidone (10ml) was heated at 140° for 22 hours. After cooling, the mixture was acidified with 0.1N hydrochloric acid and the resulting precipitate was collected by filtration. The solid was washed with chloroform to give 5-(4-hydroxyphenyl)N-methyl-N-phenylpyrimidine-2-amine (0.61g) as a white solid. This compound was dissolved in N,N-dimethylformamide (10ml) and sodium hydride (0.22g) was added. After addition of a solution of 2-diethylaminoethyl chloride hydrochloride (0.452g) in N,N-dimethylformamide (10ml) at 0°, the mixture was stirred for 4 hours at 40°. The solvent was evaporated and chloroform and water were added to the residue. The organic layer was separated and dried over magnesium sulfate. Evaporation of the solvent gave a yellow solid (0.84g), which was dissolved in chloroform and the solution was subjected to silica gel column chromatography. Elution with chloroform gave the title compound (0.69g) as a pale yellow solid. mp; 67-68.5°.

In a similar manner were prepared:-

1. N,5-Di-(4-methoxyphenyl)pyrimidine-2-amine mp 179-180°

2. 5-(4-Methoxyphenyl)-N-(4-methylphenyl)pyrimidine-2-amine mp 186-7°.

3. N-(3,4-Dichlorophenyl)-5-(4-methoxyphenyl) pyrimidine-2-amine mp 195-7°.

4. N,5-Diphenylpyrimidine-2-amine mp 171-2°.

5. 5-(4-Methylphenyl)-N-phenylpyrimidine-2-amine mp 180-1°.

6. 5-(3,4-Dichlorophenyl)-N-phenylpyrimidine-2-amine mp 206-7°.

7. N-Methyl-N-phenyl-5-(3-trifluoromethylphenyl) pyrimidine-2-amine mp 137-9°

8. N-Acetyl-5-(4-methylphenyl)-N-phenylpyrimidine-2-amine mp 74-5°.

9. N-Methyl-5-(4-methylphenyl)-N-phenylpyrimidine-2-amine mp 118-9°.

10. 5-(4-Methoxyphenyl)-N-phenyl-N-propylpyrimidine-2-amine mp 72-4°.

11. 5-(4-Dimethylaminophenyl)-N-methyl-N-phenylpyrimidine-2-amine mp 140-1°.

12. N-(4-Cyanophenyl)-5-(4-methoxyphenyl)-pyrimidine-2-amine mp 183-6°.

13. N,N-Diphenyl-5-(4-methoxyphenyl)pyrimidine-2-amine mp 141-3°.

14. Methyl 4-[5-(4-methoxyphenyl)pyrimid-2-yl] aminobenzoate mp 188-90°.

15. N-Benzyl-5-(4-methoxyphenyl)-N-phenylpyrimidine-2-

amine mp 116-8°.

16. N-(4-Acetylphenyl)-5-(4-methoxyphenyl)-pyrimidine -2-amine mp 185-7°.

17. 4-[5-(4-Methoxyphenyl)pyrimid-2-yl]aminobenzamide mp 263-5°.

18. N,N-Dimethyl-4-[2-(N-methyl-N-phenyl)amino-pyrimidin-5-yl]benzene sulphonamide mp 190-192°.

19. N-Methyl-N-phenyl-4-phenylpyrimidine-2-amine mp 88-90°.

20. 4-(4-Methoxyphenyl)-N-methyl-N-phenylpyrimidine-2-amine mp 104-106°.

21. N-Methyl-N-phenyl-5-(4-pyridyl)pyrimidine-2-amine mp 116-117.5°.

22. N-Phenyl-5-(pyrimidin-4-yl)pyrimidine-2-amine mp 238-9°.

23. 1,2-Dihydro-5-(4-methoxyphenyl)-2-phenylimino-1-propylpyrimidine mp 73-74.5°.

24. N-Methyl-N-phenyl-5-(pyrimidin-4-yl)pyrimidine-2-amine mp 127.5-8.5.

25. 5-(4-Methoxyphenyl)-2-(N-methyl-N-phenylamino) -4(1H)-pyrimidone mp 205-207°.

26. 4-Methoxy-5-(4-methoxyphenyl)-N-methyl-N-phenyl pyrimidine-2-amine mp 113-114°.

27. 5-(2-Methylphenyl)-N-phenylpyrimidine-2-amine mp 129-131°.

28. N-Methyl-5-(2-methylphenyl)-N-phenylpyrimidine-2-amine mp 88-9°.

29. 5-(4-Methoxyphenyl)-N-methyl-N-(2-methylphenyl)-pyrimidine-2-amine mp 116-117.5°.

30. 5-(4-Methoxyphenyl)-N-(2-methylphenyl)pyrimidine-2-amine mp 104-6°.

31. 5-(4-Methoxyphenyl)-4-methyl-N-phenylpyrimidine-2-amine mp 163-5°.

32. 5-(4-Methoxyphenyl)-4,N-dimethyl-N-phenylpyrimidine-2-amine mp 102-4°.

33. 4,6-Dimethoxy-5-(4-methoxyphenyl)-N-phenylpyrimidine -2-amine mp 160-2°.

34. 4,6-Dimethoxy-5-(4-methoxyphenyl)-N-methyl-N-phenylpyrimidine-2-amine mp 112-15°.

35. 5-(4-Methoxyphenyl)-N-(4-methylthiazol-2-yl)pyrimidine -2-amine mp 240-2°.

36. 5-(4-Methoxyphenyl)-N-methyl-N-(4-methylthiazol-2-yl)-pyrimidine-2-amine mp 139-41°.

37. 5-(4-Methoxyphenyl)-N-methyl-N-(pyrimidin-2-yl) pyrimidine-2-amine mp 96-8°.

38. N-Phenyl-5-(pyrazin-2-yl)pyrimidine-2-amine mp 207-8°.

39. N-Methyl-N-phenyl-5-(pyrazin-2-yl)pyrimidine-2-amine mp 129.5-130.50°.

40. 3-(2-Phenylaminopyrimidin-5-yl)pyridazine

mp 196-7°.

41. 3-[2-(N-Methyl-N-phenylamino)pyrimidin-5-yl]pyridazine mp 146-7.5°.

42. N-Ethyl-5-(4-methoxyphenyl)-N-phenylpyrimidine-2-amine mp 99.5-100.5°.

43. 1,2-Dihydro-1-ethyl-5-(4-methoxyphenyl)-2-phenylimino-pyrimidine mp 83-4°.

44. N-Phenyl-4-(4-pyridyl)pyrimidine-2-amine mp 149-50°.

45. N-Methyl-N-phenyl-4-(4-pyridyl)pyrimidine-2-amine mp 109-11°.

46. 5-(3-Methoxyphenyl)-N-methyl-N-phenylpyrimidine-2-amine mp 102-3°.

47. 5-(2-Methoxyphenyl)-N-methyl-N-phenylpyrimidine-2-amine mp 73-75°.

48. 1,2-Dihydro-2-phenylimino-1-propyl-5-(pyrimidin-4-yl)pyrimidine hydroiodide mp 235-237°.

49. 5-(1-Methylimidazol-2-yl)-N-phenylpyrimidine-2-amine mp 160-161.5°.

50. N-Methyl-5-(1-methyl-1H-imidazol-2-yl)-N-phenyl pyrimidine-2-amine mp 187.5-188.5°.

51. 5-(4-Methoxyphenyl)-N-(1-methylethyl)-N-phenyl pyrimidine-2-amine mp 112-115°.

<u>Example A</u>

<u>Augmentation of depressed immune responses</u>

Tumour bearing animals are often found to have significantly depressed immune responses. This depression is currently thought to be a result of the tumour releasing immunosuppressive factors. In man, this immunosuppression is further exacerbated by the treatment of the tumour, such as surgery, chemotherapy and radiotherapy, all of which are known to cause immunosuppression.

The compounds of the invention augment immune responses in normal animals. To ascertain if augmentation can be achieved in tumour bearing animals or drug treated animals the following Experiments are performed.

a) C57/Bl mice receive subcutaneous implantations of small pieces of Lewis lung sarcoma just above their hind legs. The compound is dosed orally at 50mg/kg daily for the next four days. One week after implantation the mice are sensitised with 5% oxazolone on their shaved abdomen and further dosed with test compound as above for the next two days. 14 days after implantation the mice are challenged on their left ears with 1% oxazolone and ear thickness increases (a measure of a delayed contact cell mediated immune response) are read 24 hours later. The delayed contact response in control mice with tumour implants alone is significantly inhibited.

b) The immune response to oxazolone in $C_{57}$Bl mice is

inhibited by the alkylating agent cyclophosphamide, given two days after sensitisation with oxazolone, at a dose of 200mg/kg by the intraperitoneal route. A 64% inhibition of the contact response was observed. In mice dosed with the compounds of the invention at 20mg/kg by the subcutaneous route, the day before, on, and the day after sensitisation, the inhibition was decreased thus showing a significant rescue from the inhibitory effect of cyclophosphamide.

0055J(ir)/jaa

What we claim is:-

1. The use as a pharmaceutical of a compound of formula I,

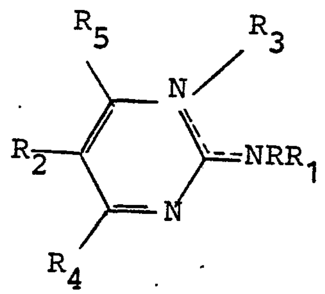

I

in which one of the groups R and $R_3$ has no significance and the other is hydrogen, phenyl or alkyl C1 to 6 optionally substituted by phenyl, and when $R_3$ has no significance R can additionally represent alkanoyl C1 to 6,

one of the bonds --- is a double bond and the other is a single bond,

$R_1$, and at least one of $R_2$, $R_4$ and $R_5$, may be the same or different and are selected from a pyridinyl, pyrimidinyl, thiazolyl, pyrazinyl, pyridazinyl, imidazolyl and phenyl ring, which rings may optionally be substituted by one or more of the groups halogen, $-COOR_5$, $-COR_5$, $-CN$, $-CONH_2$, $-SO_2NR_5R_6$, $-NR_5R_6$, $-OR_5$ or alkyl C1 to 6 which latter is optionally substituted by fluorine,

$R_5$ and $R_6$, which may be the same or different,

each represent hydrogen or alkyl C1 to 6 optionally substituted by mono- or di-alkyl (C1 to 6) amino,

and the remainder of $R_2$, $R_4$ and $R_5$ are selected from hydrogen, hydroxy, alkyl C1 to 6 and alkoxy C1 to 6 in addition to the significances given above,

or $R_1$, and one of $R_4$ and $R_5$ have the significances given above and an adjacent pair of $R_2$, $R_4$ and $R_5$ together form a -CH=CH-CH=CH- chain,

or a pharmaceutically acceptable acid addition salt thereof.

2. A compound of formula I as defined in Claim 1, or a pharmaceutically acceptable acid addition salt thereof in which $R_2$ and $R_4$ do not together form a chain -CH=CH-CH=CH-.

3. A compound according to Claim 2, wherein $R_3$ has no significance.

4. A compound according to Claim 2 or 3, wherein $R_2$ is a para alkoxy substituted benzene ring, $R_4$ and $R_5$ are both hydrogen, R is alkyl C1 to 4 or alkanoyl C2 to 4 and $R_1$ is unsubstituted phenyl.

5. A compound according to any one of Claims 2 to 4, wherein $R_2$ is a para methoxy substituted benzene ring and R is methyl or acetyl.

6. A compound according to Claim 2 which is

5-(4-Methoxyphenyl)-N-methyl-N-phenylpyrimidine-2-

amine, or

N-Acetyl-5-(4-methoxyphenyl)-N-phenylpyrimidine-2-amine.

7. A compound according to Claim 2 which is

5-(4-Methoxyphenyl)-N-phenylpyrimidine-2-amine,

1,2-Dihydro-5-(4-methoxyphenyl)-1-methyl-2-phenyliminopyrimidine,

5-(4-Methoxyphenyl)-N-(2-methylpropanoyl)-N-phenyl-pyrimidine-2-amine,

4-[5-(4-Methoxyphenyl)pyrimidin-2-yl]aminobenzoic acid,

5-(4-Hydroxyphenyl)-N-(4-hydroxyphenyl)pyrimidine-2-amine,

5-(4-Hydroxyphenyl)-N-phenylpyrimidine-2-amine,

N-Phenyl-4-(4-methoxyphenyl)pyrimidine-2-amine,

N-Phenyl-5-(4-pyridyl)pyrimidine-2-amine,

5-(4-Methoxyphenyl)-2-phenylamino-4(1H)-pyrimidinone,

4-Methoxy-5-(4-methoxyphenyl)-N-phenylpyrimidine-2-amine,

5-(4-Methoxyphenyl)-N-(2-pyrimidyl)pyrimidine-2-amine,

5-[4-(2-Diethylaminoethoxy)phenyl]-(N-methyl-N-phenylpyrimidine-2-amine,

5-(4-Hydroxyphenyl)-N-methyl-N-phenyl pyrimidine-2-amine,

N,5-Di-(4-methoxyphenyl)pyrimidine-2-amine,

5-(4-Methoxyphenyl)-N-(4-methylphenyl)pyrimidine-2-

amine,

N-(3,4-Dichlorophenyl)-5-(4-methoxyphenyl) pyrimidine-2-amine,

N,5-Diphenylpyrimidine-2-amine,

5-(4-Methylphenyl)-N-phenylpyrimidine-2-amine,

5-(3,4-Dichlorophenyl)-N-phenylpyrimidine-2-amine,

N-Methyl-N-phenyl-5-(3-trifluoromethylphenyl) pyrimidine-2-amine,

N-Acetyl-5-(4-methylphenyl)-N-phenylpyrimidine-2-amine,

N-Methyl-5-(4-methylphenyl)-N-phenylpyrimidine-2-amine,

5-(4-Methoxyphenyl)-N-phenyl-N-propylpyrimidine-2-amine,

5-(4-Dimethylaminophenyl)-N-methyl-N-phenylpyrimidine-2-amine,

N-(4-Cyanophenyl)-5-(4-methoxyphenyl)pyrimidine-2-amine,

N,N-Diphenyl-5-(4-methoxyphenyl)pyrimidine-2-amine,

Methyl 4-[5-(4-methoxyphenyl)pyrimid-2-yl] aminobenzoate,

N-Benzyl-5-(4-methoxyphenyl)-N-phenylpyrimidine-2-amine,

N-(4-Acetylphenyl)-5-(4-methoxyphenyl)pyrimidine -2-amine,

4-[5-(4-Methoxyphenyl)pyrimid-2-yl]aminobenzamide,

N,N-Dimethyl-4-[2-(N-methyl-N-phenyl)amino-pyrimidin-5-yl]benzene sulphonamide,

N-Methyl-N-phenyl-4-phenylpyrimidine-2-amine,

4-(4-Methoxyphenyl)-N-methyl-N-phenylpyrimidine-2-amine,

N-Methyl-N-phenyl-5-(4-pyridyl)pyrimidine-2-amine,

N-Phenyl-5-(pyrimidin-4-yl)pyrimidine-2-amine,

1,2-Dihydro-5-(4-methoxyphenyl)-2-phenylimino-1-propylpyrimidine,

N-Methyl-N-phenyl-5-(pyrimidin-4-yl)pyrimidine-2-amine,

5-(4-Methoxyphenyl)-2-(N-methyl-N-phenylamino)-4(1H)-pyrimidone,

4-Methoxy-5-(4-methoxyphenyl)-N-methyl-N-phenyl pyrimidine-2-amine,

5-(2-Methylphenyl)-N-phenylpyrimidine-2-amine,

N-Methyl-5-(2-methylphenyl)-N-phenylpyrimidine-2-amine,

5-(4-Methoxyphenyl)-N-methyl-N-(2-methylphenyl)-pyrimidine-2-amine,

5-(4-Methoxyphenyl)-N-(2-methylphenyl)pyrimidine-2-amine,

5-(4-Methoxyphenyl)-4-methyl-N-phenylpyrimidine-2-amine,

5-(4-Methoxyphenyl)-4,N-dimethyl-N-phenylpyrimidine-2-amine,

4,6-Dimethoxy-5-(4-methoxyphenyl)-N-phenylpyrimidine-2-amine,

4,6-Dimethoxy-5-(4-methoxyphenyl)-N-methyl-N-phenylpyrimidine-2-amine,

5-(4-Methoxyphenyl)-N-(4-methylthiazol-2-yl)pyrimidine-2-amine,

5-(4-Methoxyphenyl)-N-methyl-N-(4-methylthiazol-2-yl)-pyrimidine-2-amine,

5-(4-Methoxyphenyl)-N-methyl-N-(pyrimidin-2-yl) pyrimidine-2-amine,

N-Phenyl-5-(pyrazin-2-yl)pyrimidine-2-amine,

N-Methyl-N-phenyl-5-(pyrazin-2-yl)pyrimidine-2-amine,

3-(2-Phenylaminopyrimidin-5-yl)pyridazine,

3-[2-(N-Methyl-N-phenylamino)pyrimidin-5-yl]pyridazine,

N-Ethyl-5-(4-methoxyphenyl)-N-phenylpyrimidine-2-amine,

1,2-Dihydro-1-ethyl-5-(4-methoxyphenyl)-2-phenyliminopyrimidine,

N-Phenyl-4-(4-pyridyl)pyrimidine-2-amine,

N-Methyl-N-phenyl-4-(4-pyridyl)pyrimidine-2-amine,

5-(3-Methoxyphenyl)-N-methyl-N-phenylpyrimidine-2-amine,

5-(2-Methoxyphenyl)-N-methyl-N-phenylpyrimidine-2-amine,

1,2-Dihydro-2-phenyl-1-propyl-5-(pyrimidin-4-yl)iminopyrimidine hydroiodide,

5-(1-Methylimidazol-2-yl)-N-phenylpyrimidine-2-amine,

N-Methyl-5-(1-methyl-1H-imidazol-2-yl)-N-phenyl-

0164204



pyrimidine-2-amine, or

5-(4-Methoxyphenyl)-N-(1-methylethyl)-N-phenyl pyrimidine-2-amine.

8. A pharmaceutical composition comprising a compound according to any one of Claims 2 to 7 and a pharmaceutically acceptable adjuvant, diluent or carrier.

9. The use of a compound according to any one of Claims 2 to 8 for the manufacture of a medicament for the treatment of a patient haveing depressed immune responses.

10. A process for the production of a compound according to Claim 2, which comprises

a) reaction of a compound of formula II,

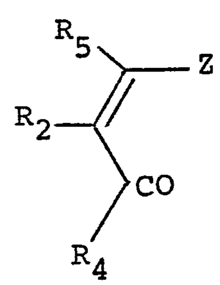

II

in which $R_2$, $R_4$ and $R_5$ are as defined above, and Z is a good leaving group,

with a compound of formula III,

$$R_1\text{-NRC}(=NR_3)NH_2 \qquad III$$

or a salt thereof,

in which R, $R_1$ and $R_3$ are as defined above,

b) production of a compound of formula I in which R is alkanoyl $C_1$ to 6 by alkanoylation of a corresponding compound of formula I in which one of R and $R_3$ is hydrogen,

c) production of a compound of formula I in which one of R and $R_3$ is alkyl C1 to 6 optionally substituted by phenyl, by alkylation or phenylalkylation of a corresponding compound of formula I in which one of R and $R_3$ is hydrogen,

d) production of a compound of formula I carrying a -COOH substituent by hydrolysis of a corresponding compound of formula I carrying a -COOalkyl or $-CONH_2$ substituent,

e) production of a compound of formula I carrying an -OH substituent by removal of an alkyl group from a corresponding compound of formula I carrying an alkoxy substituent, or

f) production of a compound of formula I in which at least one of $R_2$, $R_4$ and $R_5$ is alkoxy optionally substituted by mono-or di-alkylamino, by reaction of a corresponding compound of formula I in which at least one of $R_2$, $R_4$ and $R_5$ is hydroxy, with an optionally mono-or di-alkylamino substituted alkylating agent,

and if desired or necessary converting the resulting compound of formula I to pharmaceutically acceptable salt

thereof or vice versa.

0055J/jed

What we claim is:-

1. A process for the production of a compound of formula I,

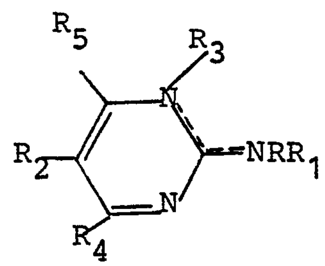

I

in which one of the groups R and $R_3$ has no significance and the other is hydrogen, phenyl or alkyl C1 to 6 optionally substituted by phenyl, and when $R_3$ has no significance R can additionally represent alkanoyl C1 to 6,

one of the bonds --- is a double bond and the other is a single bond,

$R_1$, and at least one of $R_2$, $R_4$ and $R_5$, may be the same or different and are selected from a pyridinyl, pyrimidinyl, thiazolyl, pyrazinyl, pyridazinyl, imidazolyl and phenyl ring, which rings may optionally be substituted by one or more of the groups halogen, $-COOR_5$, $-COR_5$, -CN, $-CONH_2$, $-SO_2NR_5R_6$, $-NR_5R_6$, $-OR_5$ or alkyl C1 to 6 which latter is optionally substituted by fluorine,

$R_5$ and $R_6$, which may be the same or different, each represent hydrogen or alkyl C1 to 6 optionally substituted by mono- or di-alkyl (C1 to 6) amino,

and the remainder of $R_2$, $R_4$ and $R_5$ are selected from hydrogen, hydroxy, alkyl C1 to 6 and alkoxy C1 to 6 in addition to the significances given above,

or a pharmaceutically acceptable acid addition salt thereof,

which comprises

a) reaction of a compound of formula II,

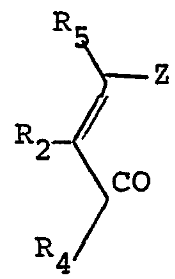

II

in which $R_2$, $R_4$ and $R_5$ are as defined above, and Z is a good leaving group,

with a compound of formula III,

$$R_1\text{-NRC}(=NR_3)NH_2 \qquad \text{III}$$

or a salt thereof,

in which R, $R_1$ and $R_3$ are as defined above,

b) production of a compound of formula I in which R is alkanoyl C1 to 6 by alkanoylation of a corresponding

compound of formula I in which one of R and $R_3$ is hydrogen,

c) production of a compound of formula I in which one of R and $R_3$ is alkyl C1 to 6 optionally substituted by phenyl, by alkylation or phenylalkylation of a corresponding compound of formula I in which one of R and $R_3$ is hydrogen,

d) production of a compound of formula I carrying a -COOH substituent by hydrolysis of a corresponding compound of formula I carrying a -COOalkyl or $-CONH_2$ substituent,

e) production of a compound of formula I carrying an -OH substituent by removal of an alkyl group from a corresponding compound of formula I carrying an alkoxy substituent, or

f) production of a compound of formula I in which at least one of $R_2$, $R_4$ and $R_5$ is alkoxy optionally substituted by mono-or di-alkylamino, by reaction of a corresponding compound of formula I in which at least one of $R_2$, $R_4$ and $R_5$ is hydroxy, with an optionally mono-or di-alkylamino substituted alkylating agent,

and if desired or necessary converting the resulting compound of formula I to pharmaceutically acceptable salt thereof or vice versa.

2. A process according to Claim 1, wherein $R_3$ has no

significance.

3. A process according to Claim 1 or 2, wherein $R_2$ is a para alkoxy substituted benzene ring, $R_4$ and $R_5$ are both hydrogen, R is alkyl C1 to 4 or alkanoyl C2 to 4 and $R_1$ is unsubstituted phenyl.

4. A process according to any one of Claims 1 to 3, wherein $R_2$ is a para methoxy substituted benzene ring and R is methyl or acetyl.

5. A process according to Claim 1, wherein the compound of formula I is

5-(4-Methoxyphenyl)-N-methyl-N-phenylpyrimidine-2-amine, or

N-Acetyl-5-(4-methoxyphenyl)-N-phenylpyrimidine-2-amine.

6. A process according to Claim 1, wherein the compound of formula I is

5-(4-Methoxyphenyl)-N-phenylpyrimidine-2-amine,

1,2-Dihydro-5-(4-methoxyphenyl)-1-methyl-2-phenyliminopyrimidine,

5-(4-Methoxyphenyl)-N-(2-methylpropanoyl)-N-phenyl-pyrimidine-2-amine,

4-[5-(4-Methoxyphenyl)pyrimidin-2-yl]aminobenzoic acid,

5-(4-Hydroxyphenyl)-N-(4-hydroxyphenyl)pyrimidine-2-amine,

5-(4-Hydroxyphenyl)-N-phenylpyrimidine-2-amine,

N-Phenyl-4-(4-methoxyphenyl)pyrimidine-2-amine,

N-Phenyl-5-(4-pyridyl)pyrimidine-2-amine,

5-(4-Methoxyphenyl)-2-phenylamino-4(1H)-pyrimidinone,

4-Methoxy-5-(4-methoxyphenyl)-N-phenylpyrimidine -2-amine,

5-(4-Methoxyphenyl)-N-(2-pyrimidyl)pyrimidine-2-amine,

5-[4-(2-Diethylaminoethoxy)phenyl]-(N-methyl-N-phenylpyrimidine-2-amine,

5-(4-Hydroxyphenyl)-N-methyl-N-phenyl pyrimidine-2-amine,

N,5-Di-(4-methoxyphenyl)pyrimidine-2-amine,

5-(4-Methoxyphenyl)-N-(4-methylphenyl)pyrimidine-2-amine,

N-(3,4-Dichlorophenyl)-5-(4-methoxyphenyl) pyrimidine-2-amine,

N,5-Diphenylpyrimidine-2-amine,

5-(4-Methylphenyl)-N-phenylpyrimidine-2-amine,

5-(3,4-Dichlorophenyl)-N-phenylpyrimidine-2-amine,

N-Methyl-N-phenyl-5-(3-trifluoromethylphenyl) pyrimidine-2-amine,

N-Acetyl-5-(4-methylphenyl)-N-phenylpyrimidine-2-amine,

N-Methyl-5-(4-methylphenyl)-N-phenylpyrimidine-2-amine,

5-(4-Methoxyphenyl)-N-phenyl-N-propylpyrimidine-2-

amine,

5-(4-Dimethylaminophenyl)-N-methyl-N-phenylpyrimidine-2-amine,

N-(4-Cyanophenyl)-5-(4-methoxyphenyl)pyrimidine-2-amine,

N,N-Diphenyl-5-(4-methoxyphenyl)pyrimidine-2-amine,

Methyl 4-[5-(4-methoxyphenyl)pyrimid-2-yl]aminobenzoate,

N-Benzyl-5-(4-methoxyphenyl)-N-phenylpyrimidine-2-amine,

N-(4-Acetylphenyl)-5-(4-methoxyphenyl)pyrimidine-2-amine,

4-[5-(4-Methoxyphenyl)pyrimid-2-yl]aminobenzamide,

N,N-Dimethyl-4-[2-(N-methyl-N-phenyl)aminopyrimidin-5-yl]benzene sulphonamide,

N-Methyl-N-phenyl-4-phenylpyrimidine-2-amine,

4-(4-Methoxyphenyl)-N-methyl-N-phenylpyrimidine-2-amine,

N-Methyl-N-phenyl-5-(4-pyridyl)pyrimidine-2-amine,

N-Phenyl-5-(pyrimidin-4-yl)pyrimidine-2-amine,

1,2-Dihydro-5-(4-methoxyphenyl)-2-phenylimino-1-propylpyrimidine,

N-Methyl-N-phenyl-5-(pyrimidin-4-yl)pyrimidine-2-amine,

5-(4-Methoxyphenyl)-2-(N-methyl-N-phenylamino)-4(1H)-pyrimidone,

4-Methoxy-5-(4-methoxyphenyl)-N-methyl-N-phenyl pyrimidine-2-amine,

5-(2-Methylphenyl)-N-phenylpyrimidine-2-amine,

N-Methyl-5-(2-methylphenyl)-N-phenylpyrimidine-2-amine,

5-(4-Methoxyphenyl)-N-methyl-N-(2-methylphenyl)-pyrimidine-2-amine,

5-(4-Methoxyphenyl)-N-(2-methylphenyl)pyrimidine-2-amine,

5-(4-Methoxyphenyl)-4-methyl-N-phenylpyrimidine-2-amine,

5-(4-Methoxyphenyl)-4,N-dimethyl-N-phenylpyrimidine-2-amine,

4,6-Dimethoxy-5-(4-methoxyphenyl)-N-phenylpyrimidine -2-amine,

4,6-Dimethoxy-5-(4-methoxyphenyl)-N-methyl-N-phenylpyrimidine-2-amine,

5-(4-Methoxyphenyl)-N-(4-methylthiazol-2-yl)pyrimidine -2-amine,

5-(4-Methoxyphenyl)-N-methyl-N-(4-methylthiazol-2-yl)-pyrimidine-2-amine,

5-(4-Methoxyphenyl)-N-methyl-N-(pyrimidin-2-yl) pyrimidine-2-amine,

N-Phenyl-5-(pyrazin-2-yl)pyrimidine-2-amine,

N-Methyl-N-phenyl-5-(pyrazin-2-yl)pyrimidine-2-amine,

3-(2-Phenylaminopyrimidin-5-yl)pyridazine,

3-[2-(N-Methyl-N-phenylamino)pyrimidin-5-yl]pyridazine,

N-Ethyl-5-(4-methoxyphenyl)-N-phenylpyrimidine-2-amine,

1,2-Dihydro-1-ethyl-5-(4-methoxyphenyl)-2-phenyliminopyrimidine,

N-Phenyl-4-(4-pyridyl)pyrimidine-2-amine,

N-Methyl-N-phenyl-4-(4-pyridyl)pyrimidine-2-amine,

5-(3-Methoxyphenyl)-N-methyl-N-phenylpyrimidine-2-amine,

5-(2-Methoxyphenyl)-N-methyl-N-phenylpyrimidine-2-amine,

1,2-Dihydro-2-phenyl-1-propyl-5-(pyrimidin-4-yl)iminopyrimidine hydroiodide,

5-(1-Methylimidazol-2-yl)-N-phenylpyrimidine-2-amine,

N-Methyl-5-(1-methyl-1H-imidazol-2-yl)-N-phenylpyrimidine-2-amine, or

5-(4-Methoxyphenyl)-N-(1-methylethyl)-N-phenyl pyrimidine-2-amine.

7. The use of a compound of formula I as defined in any one of Claims 1 to 6, or a pharmaceutically acceptable salt thereof, for the manufacture of a medicament for the treatment of a patient having depressed immune responses.

0364J/jed

0164204

European Patent Office

# EUROPEAN SEARCH REPORT

Application number

EP 85302902.3

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | DE - A1 -3 220 118 (BAYER AG)<br>* Claims 1-3, 5-8 *<br>-- | 1-3,7-9 | A 61 K 31/505<br>C 07 D 401/12<br>C 07 D 403/12<br>C 07 D 417/12<br>C 07 D 239/42<br>C 07 D 401/04<br>C 07 D 403/04<br>C 07 D 417/04 |
| A | US - A - 4 010 269 (H.E. RENIS, L.L. SKALETZKY)<br>* Claim 1; column 1, lines 15-58 *<br>-- | 1,8,9 | |
| A | CA - A - 1 020 942 (THE UPJOHN COMPANY)<br>* Page 2, line 17 - page 4, line 16 *<br>-- | 1,8,9 | |
| A | US - A - 3 663 706 (H.-J. E. HESS)<br>* Column 1, line 30 - column 2, line 66 *<br>-- | 1,8,9 | **TECHNICAL FIELDS SEARCHED (Int. Cl.4)** |
| A | GB - A - 1 176 854 (CHAS. PFIZER & CO., INC.)<br>* Page 1, line 26 - page 2, line 127 *<br>-- | 1,8,9 | A 61 K 31/00<br>C 07 D 239/00<br>C 07 D 277/00<br>C 07 D 401/00<br>C 07 D 403/00<br>C 07 D 417/00 |
| A | DE - A - 2 051 430 (ELI LILLY)<br>* Claim 1 *<br>---- | 1,8,9 | |

The present search report has been drawn up for all claims

| Place of search | Date of completion of the search | Examiner |
|---|---|---|
| VIENNA | 29-08-1985 | MAZZUCCO |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03 82